# EUROPEAN PATENT SPECIFICATION

(11) **EP 2 635 293 B1**
(45) Date of publication and mention of the grant of the patent: **04.04.2018**
(21) Application number: 10859369.0
(22) Date of filing: 03.11.2010
(51) Int. Cl.: A61K 38/16, A61K 9/06, A61K 9/70, A61P 17/02, A61P 17/00

(54) **SKIN WOUND HEALING COMPOSITIONS AND METHODS OF USE THEREOF**
HEILUNGSZUSAMMENSETZUNGEN FÜR HAUTWUNDEN UND VERWENDUNGSVERFAHREN DAFÜR
COMPOSITIONS DE CICATRISATION DE PLAIES CUTANÉES ET LEURS PROCÉDÉS D'UTILISATION

(43) Date of publication of application: 11.09.2013
(73) Proprietor: University of Southern California, Los Angeles, CA 90015 (US)
(72) Inventor: CHENG, Chieh-Fang, Mountain View, California 94040 (US); WOODLEY, David, T., Altadena, CA 91001 (US); CHEN, Mei, Atladena, CA 91001 (US); LI, Wei, Altadena, CA 91001 (US)
(74) Representative: Müller-Boré & Partner Patentanwälte PartG mbB
(86) International application number: PCT/US2010/055319
(87) International publication number: WO 2012/060832

(56) References cited:
- WO-A1-00/23093
- WO-A1-92/03476
- WO-A1-92/03476
- WO-A1-2007/083853
- WO-A1-2008/086358
- US-A1- 2007 098 735
- US-A1- 2009 005 317
- US-A1- 2009 305 973
- JOSEPH T. KOVALCHIN ET AL: "In vivo delivery of heat shock protein 70 accelerates wound healing by up-regulating macrophage-mediated phagocytosis", WOUND REPAIR AND REGENERATION, vol. 14, no. 2, 1 March 2006 (2006-03-01), pages 129-137, XP055102539, ISSN: 1067-1927, DOI: 10.1111/j.1743-6109.2006.00102.x
- LI WEI ET AL: "Extracellular heat shock protein-90alpha: linking hypoxia to skin cell motility and wound healing.", THE EMBO JOURNAL 7 MAR 2007, vol. 26, no. 5, 7 March 2007 (2007-03-07), pages 1221-1233, XP002720702, ISSN: 0261-4189
- SONG X ET AL: "The regulatory mechanism of Hsp90alpha secretion from endothelial cells and its role in angiogenesis during wound healing", BIOCHEMICAL AND BIOPHYSICAL RESEARCH COMMUNICATIONS, ACADEMIC PRESS INC. ORLANDO, FL, US, vol. 398, no. 1, 16 July 2010 (2010-07-16) , pages 111-117, XP027298201, ISSN: 0006-291X, DOI: 10.1016/J.BBRC.2010.06.046 [retrieved on 2010-06-15]
- LAPLANTE A F ET AL: "Expression of heat shock proteins in mouse skin during wound healing.", THE JOURNAL OF HISTOCHEMISTRY AND CYTOCHEMISTRY : OFFICIAL JOURNAL OF THE HISTOCHEMISTRY SOCIETY NOV 1998, vol. 46, no. 11, November 1998 (1998-11), pages 1291-1301, XP002720703, ISSN: 0022-1554
- S. D. MORRIS: "Heat shock proteins and the skin", CLINICAL AND EXPERIMENTAL DERMATOLOGY, vol. 27, no. 3, 1 May 2002 (2002-05-01), pages 220-224, XP055102544, ISSN: 0307-6938, DOI: 10.1046/j.1365-2230.2002.01012.x
- CHENG C -F ET AL: "A fragment of secreted Hsp90[alpha] carries properties that enable it to accelerate effectively both acute and diabetic wound healing in mice", JOURNAL OF CLINICAL INVESTIGATION 20111101 THE JOURNAL OF CLINICAL INVESTIGATION USA, vol. 121, no. 11, 1 November 2011 (2011-11-01), pages 4348-4361, XP002720704, ISSN: 0021-9738

## Description

The present invention relates to wound healing compositions and uses thereof. Particularly, the present invention relates to compositions of polypeptides and the topical application of these compositions to the skin to expedite wound healing by promoting all skin cell migration. The invention is defined by the claims.

Wound healing, or wound repair, is an intricate process in which the skin repairs itself after injury. In normal skin, the epidermis (outermost layer) and dermis (inner or deeper layer) exists in a steady-stated equilibrium, forming a protective barrier against the external environment. The normal wound healing process can be broadly classified into three stages namely the inflammatory, proliferative and maturation phases. The inflammatory phase lasts 0-2 days and involves an orderly recruitment of cells to the wound area. This is followed by the 2-6 day proliferative phase, in which fibroblasts, keratinocytes and other cells in the wound bed begin to actively proliferate to close the wound. During the first phase of tissue repair, an acute inflammatory response with cellular migration occurs. Neutrophils predominate for the first 24-48 hours; macrophages become active by the third day. The neutrophils and macrophages phagocytose and digest pathologic organisms and tissue debris. The maturation phase follows the proliferative phase, peaking at 21 days, by which time the wound is completely healed by restructuring the initial scar tissue.

A problematic wound does not follow the normal time table for the healing process as described above. The increased time required for a problematic wound can cause unwanted cost and pain associated with the slowed healing, as well as a decrease in job production and overall quality of life. Among the two million people diagnosed yearly with pressure ulcers, 900,000 have non-healing lower extremity ulcers. It is estimated that 18% of patients with diabetes over the age of 65 will have chronic, non-healing foot ulcers. Moreover, 50,000 lower extremity amputations are performed each year due to infected lower leg chronic wounds. The quality of life due to morbidity of non-healing leg ulcers is significantly compromised because of wound odor, infection, and pain. In addition, these issues also lead to social isolation and diminished self-image in patients with chronic skin wounds. Financially, the cost for managing delayed wound healing in the US elderly is estimated at $9 billion per year.

A great deal of time and expense has been utilized in the field of chronic wound healing. Akella et al. discloses in US Pat. No. 7,081,240, the use of a protein mixture for treating wounds, wherein the mixture is isolated from bone or produced from recombinant proteins such as bone morphogenetic proteins, transforming growth factors and fibroblast growth factors. However, the overall clinical outcomes of growth factor therapy have been disappointing and few growth factors have ultimately received FDA approval.

Kiss discusses the use of non-growth factor proteins for use in wound healing comprised of human alpha1-antitrypsin, human placental alkaline phosphatase, human transferring and α₁-acid glycoprotein. However, this method's draw back is that it requires the complicated sequential application of several agents that act at different steps, and also may require adjustment of the compositions according to each treatment. Similarly, the use of skin substitutes has not been cost-effective.

Re-epithelialization is a critical event in human skin wound healing, in which epidermal keratinocytes laterally migrate to close a wound. In chronic wounds, keratinocyte migration is blocked and the wounds remain open, causing patient morbidity and even fatality.

During human skin wound healing, a critical rate-limiting step is the initiation of the resident epidermal and dermal cells at the wound edge to migrate into the wound bed. Human keratinocytes (HKCs) laterally migrate across the wound bed from the cut edge to eventually close the wound, the process known as re-epithelialization. The dermal cells, including dermal fibroblasts (DFs) and dermal microvascular endothelial cells (HDMECs), start to move into the wound following the HKC migration, where these cells deposit matrix proteins, contract and remodel the newly closed wound and build new blood vessels. HKC migration is largely driven by TGFα in human serum and is not affected by high concentrations of TGFβ family cytokines co-present in human serum. In contrast, the presence of TGFβ blocks the dermal cell migration even in the presence of their growth factors, such as PDGF-BB and VEGF. Therefore, while it is understandable why HKC migration jumpstarts ahead of DF and HDMEC migration during wound healing, it has remained as a puzzle how DFs and HDMECs move into the wound bed in the presence of abundant TGFβ.

Other research has involved the use of heat shock protein to promote wound healing. For example, Srivastava et al. discloses in US Pat. No. 6,475,490 compositions comprising heat shock proteins, including gp96, hsp90, and hsp70, uncomplexed or complexed noncovalently with antigenic molecules. However, the use of the entire length of these large molecules in pharmaceutical compositions results a reduced efficacy per unit weight of the protein.

In this context, Li *et al.* (Li, W. et al.; Extracellular heat shock protein-90α: linking hypoxia to skin motility and wound healing; The EMBO Journal, 26(5); 2007; pp. 1221-1233) describes hsp90a and its use for wound healing. Further, Laplante *et al.* (Laplante, A. F. et al.; Expression of Heat Shock Proteins in Mouse Skin During Wound Healing; J Histochem Cytochem, 46(11); 1998; pp. 1291-1302) describes the expression of heat shock proteins in mouse skin during wound healing.
In order to overcome the above mentioned problems, the present invention identifies a wound healing composition comprising a polypeptide compound having an hsp90a polypeptide chain, wherein the polypeptide chain consists of the amino acid sequences EEKEDKEEEKEKEEKESEDKPEIEDVGSDEEEEKKDGDKKKKKKIKEKYIDQEE or SDEEEEKKDGDKKKKKKIKEKYIDQEE.

Optionally, the composition includes a pharmaceutical medium to carry the polypeptide compound, such as an aqueous solution, suspension, dispersion, salve, ointment, gel, cream, lotion, spray or paste.

Optionally, the composition can comprise a mixture of polypeptide chains of from 5 to 120 amino acid units, 20-60 amino acid units or the specific amino acid units shown above.

The present invention is also directed to the above wound healing composition for use in a method of healing a skin wound.

In another embodiment, the polypeptide compound is formulated in a concentration of from 10 µg/ml to 3 mg/ml in said pharmaceutical medium. Optionally, the polypeptide compound is formulated in a concentration of from 30 µg/ml to 500 µg/ml in said pharmaceutical medium.

In one embodiment, the composition is applied to the wound every 6 to every 72 hours. Optionally, the composition is applied to the wound every 24 to every 48 hours.

Additional advantages and other features of the present disclosure will be set forth in part in the description which follows and in part will become apparent to those having ordinary skill in the art upon examination of the following or may be learned from the practice of the disclosure. The advantages of the disclosure may be realized and obtained as particularly pointed out in the appended claims.

The figures show:
Fig. 1 is a bar graph showing various efficacies of polypeptide compounds and their effects on wound healing in mice as compared to a control cream.
Figs. 2a-f are pictures of the results of skin wound healing of mice comparing a polypeptide having a peptide chain with 115 amino acids vs a control cream.
Figs. 3a-3e are pictures of the results of skin wound healing of mice comparing an FDA approved compound (Regranex ™, PDGF-BB) vs a control cream.

Not to be bound by theory, it is believed that following skin injury, paracrine- or autocrine-released TGFα stimulates membrane translocation and secretion of pre-existing hsp90α proteins in HKCs. The secreted hsp90α jumpstarts HKC migration, a critical event of re-epithelialization process, by binding to the CD91/LRP-1 receptor on the cell surface. When extracellular hsp90α defuses into and reached certain concentration in the wound bed, it starts to induce migration of DFs and HDMECs from the cut edge into the wound bed even under "hazard" conditions: no ATP and ATPase activity and in presence of general cell motility inhibitors, such as TGFβ family cytokines. Thus, extracellular hsp90α is utilized for skin wound healing. However, when chronic wounds are present, the skin is unable to produce hsp90α for wound healing. Thus, an additive to promote skin wound healing must be applied to the wound.

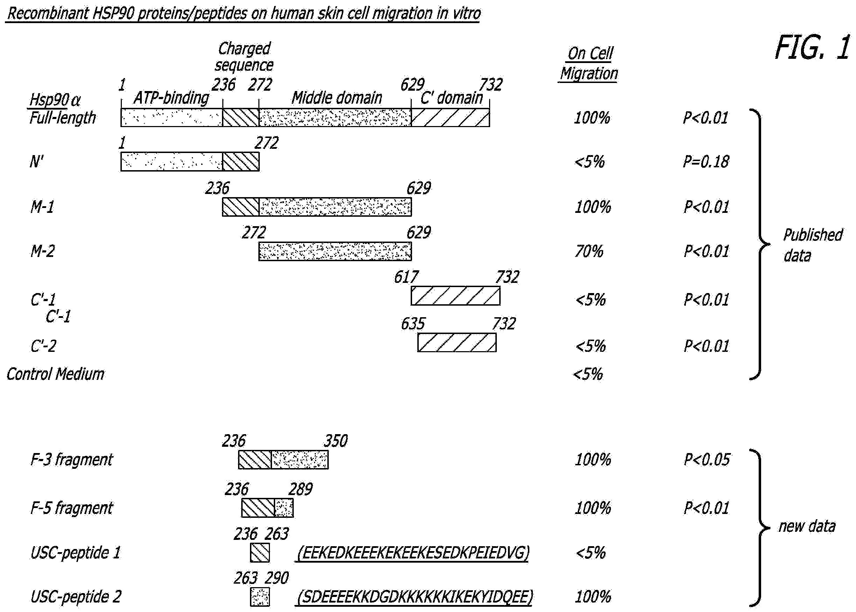

As can be seen in **Fig. 1****,** the full-length (WT) hsp90α showed a remarkable pro-motility activity, in comparison to the control medium. The middle domain plus the charged sequence (M-1) show a similar degree of the activity as the WT hsp90α. However, the middle domain lacking the charged sequence showed a significantly decreased activity (M-2), although the charged sequence plus the entire N'-terminal domain (N') showed no stimulating activity. The two C'-terminal domains (C'-1 and C'-2, made to ensure the results) both showed a moderate pro-motility activity. Therefore, hsp90α promotes HKC migration mainly through its middle and the carboxyl domains, consistent with their surface location in hsp90α. Accordingly, a composition comprising the middle domain of hsp90α would exhibit similar wound healing properties as that of a composition comprising hsp90α alone.

However, as can also be seen in **Fig. 1**, not all of the full hsp90α protein or the entire middle domain and charged portion of hsp90α protein is necessary to promote wound healing. The F-3 fragment consisting of 115 amino acid units which is from amino acid number 236 to 350 and the F-5 fragment having 54 amino acid units from amino acid number 236 to 289 (sequence EEKEDKEEEKEKEEKESEDKPEIEDVGS DEEEEKKDGDKKKKKKIKEKYIDQEE) exhibit similar activity as that of full length hsp90α, that being 100% on cell migration. Thus, the unnecessary portions of hsp90α need not be included in a composition for wound healing, thereby allowing a composition to be more efficacious per weight of active wound healing agent. Moreover, instead of relying on complicated isolation and extraction methods to obtain the hsp90a protein or the middle domain plus charged portion of hsp90a protein, more inexpensive and conventional methods of synthesis may be employed to obtain the lower chain length polypeptide chains.

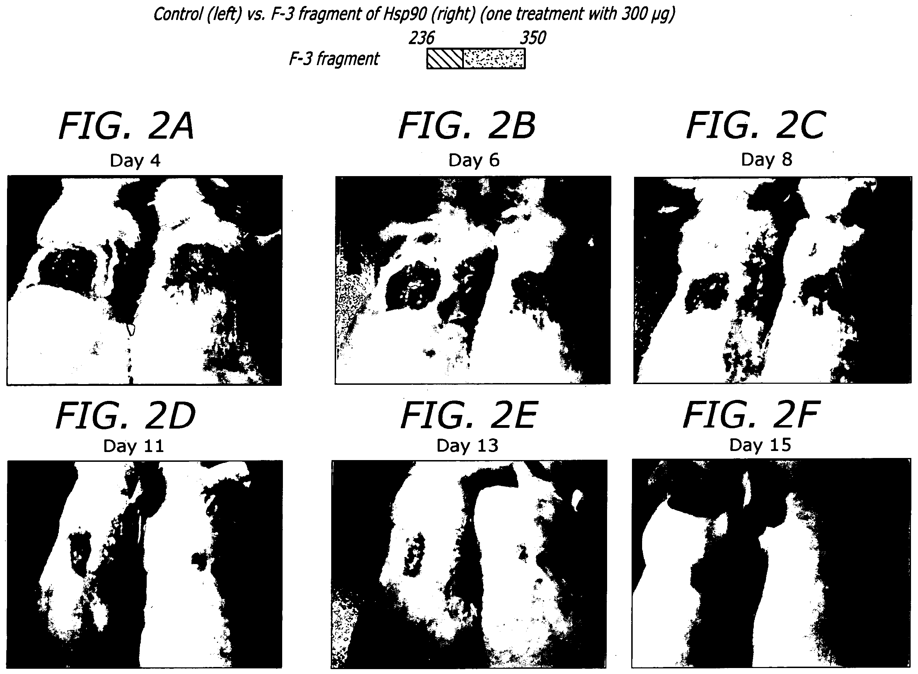

To prove the efficacy of polypeptide compounds of 115 amino acid units and 54 amino acid units were synthesized via convention means. 100 µg F-3 fragments in 100 µl of 10% Carboxymethylcellulose cream and the cream alone was topically applied to the lcm x lcm wound on the back of nude mice daily for 5 days, and wounds were analyzed every two days. Selected wound images of a representative experiment are shown in **Figs. 2A-2F****.** It can be seen that F-3 significantly accelerated closure of the wounds beginning on day 4, day 6, day 8, day 11, day 13 and day 15 as compared to the cream control.

### EXAMPLE

In the example below, the following conditions or methods were utilized.

The pharmaceutical 100µl of 10% carboxymethylcellulose cream (Sterile) is mixed in and the 1 cm x 1 cm wound on the back of a nude mouse is topically covered. Following this treatment, the wound is covered with a few antibiotics and bandi and the bandi are fixed by rolling the mouse with coban. The F-3 compound mixture is then added every day for up to five days and the wound is analyzed every two days.

To prepare mice for topical treatment of F-3, 1.0-cm x 1.0-cm full-thickness excision wounds were made by lifting the skin with forceps and removing full thickness skin with a pair of scissors on the mid-back of 8 to 10 week old mice. The wounds were topically covered by 100 µl 10% carboxymethylcellulose either without (as a control) or with 300 µg recombinant F-3 compound. The wound area was then covered with Band-Aid and Coban, a self-adherent wrap, to prevent desiccation. Only one dose of F-3 compounds was administered to the wound. To measure the wound area, standardized digital photographs were taken of the wounds at 4, 6, 8, 11, 13 and 15 days post-wounding and the open wound areas were determined with an image analyzer (AlphaEase FC version 4.1.0, Alpha Innotech Corporation). Percentage of wound area was defined by comparing areas of healing wounds to those of the original wounds. The Student T test was used for the statistical analysis. All animal studies were conducted using protocols approved by the University of Southern California Institutional Animal Use Committee.

A 1.0-cm² (1 cm x 1 cm) square full-thickness excision wound was made on the mid-back of 8 to 10 week old athymic nude mice and the pharmaceutical composition of F-3 was applied topically daily for 5 days (n = 10 mice per group). (A) Representative day 4, 6, 8, 11, 13 and 15 wounds are shown. Wound sizes were significantly reduced in mice topically treated with the cream containing F-3 (right panels), but not cream alone (left panels). (B) Mean ± SD wound size measurements at day 4, 6, 8, 11, 13 and 15 post-wounding (n = 10 mice for each group).

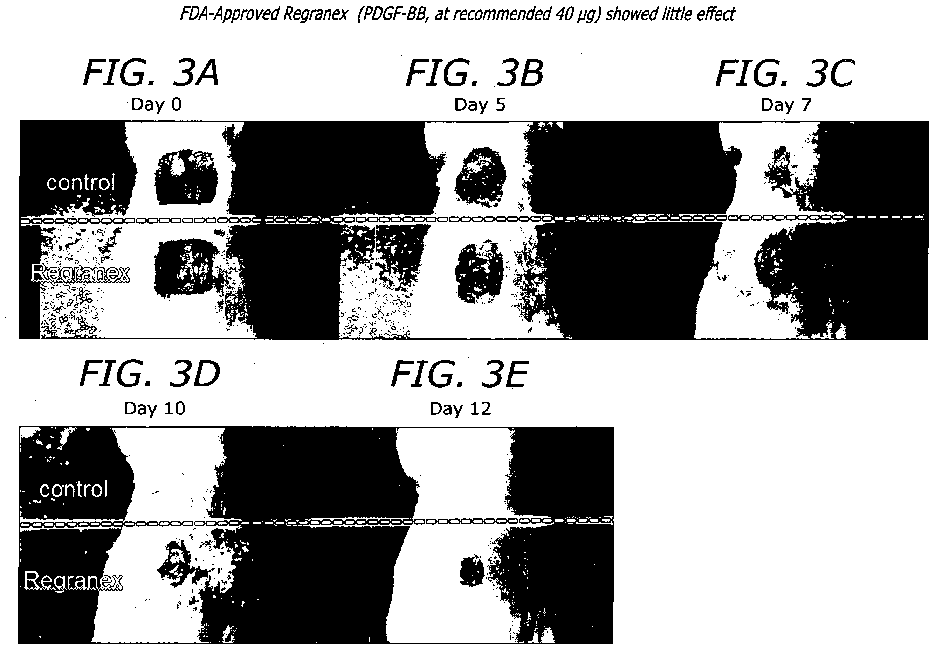

To compare efficacy of the F-3 compound, a study using FDA approved Regenerex ™ was conducted. Using the above methods, a 40 µg dose of PDGF-BB (Regenerex ™) was applied to mice for 5 days (n = 10 mice per group) vs F-3 compound. (A) Representative day 0, 5, 7, 10 and 12 wounds are shown in Figs. 3A-3E. As can be seen from the figures, wound sizes were significantly reduced in mice topically treated with the cream containing F-3 (upper panels), but not Regenerex ™ (lower panels).

The present disclosure can be practiced by employing conventional materials, methodology and equipment. Accordingly, the details of such materials, equipment and methodology are not set forth herein in detail. In the previous descriptions, numerous specific details are set forth, such as specific materials, structures, chemicals, processes, etc., in order to provide a thorough understanding of the disclosure. In other instances, well known processing structures have not been described in detail, in order not to unnecessarily obscure the present disclosure.

## Claims

1. A wound healing composition comprising:
a polypeptide compound having an hsp90α polypeptide chain,
wherein the polypeptide chain consists of the amino acid sequence
EEKEDKEEEKEKEEKESEDKPEIEDVGSDEEEEKKDGDKKKKKKIKEKYIDQEE (SEQ ID NO: 1).

2. A wound healing composition comprising:
a polypeptide compound having an hsp90α polypeptide chain,
wherein the polypeptide chain consists of the amino acid sequence
SDEEEEKKDGDKKKKKKIKEKYIDQEE
(SEQ ID NO: 2).

3. The wound healing composition according to claim 1 or claim 2, further comprising a pharmaceutical medium to carry the polypeptide compound, wherein the pharmaceutical medium is at least one selected from the group consisting of: an aqueous solution, suspension, dispersion, salve, ointment, gel, cream, lotion, spray or paste.

4. A wound healing composition according to any one of claims 1 to 3 for use in healing a skin wound.

5. The wound healing composition for use according to claim 4, wherein the polypeptide compound is formulated in a concentration of from 10 µg/ml to 3 mg/ml in said pharmaceutical medium.

6. The wound healing composition for use according to claim 4, wherein the polypeptide compound is formulated in a concentration of from 30 µg/ml to 500 µg/ml in said pharmaceutical medium.

7. The wound healing composition for use according to claim 4, wherein the composition is applied to the wound every 6 to every 72 hours.

8. The wound healing composition for use according to claim 4, wherein the composition is applied to the wound every 24 to every 48 hours.

## Patentansprüche

1. Wundheilungszusammensetzung, umfassend:
eine Polypeptidverbindung, die eine Hsp90α-Polypeptidekette aufweist,
wobei die Polypeptidkette aus der Aminosäuresequenz EEKEDKEEEKEKEEKESEDKPEIEDVGSDEEEEKKDGDKKKKKKIKEKYIDQEE (SEQ ID NO: 1) besteht.

2. Wundheilungszusammensetzung, umfassend:
eine Polypeptidverbindung, die eine Hsp90α-Polypeptidekette aufweist,
wobei die Polypeptidkette aus der Aminosäuresequenz SDEEEEKKDGDKKKKKKIKEKYIDQEE
(SEQ ID NO: 2) besteht.

3. Wundheilungszusammensetzung nach Anspruch 1 oder Anspruch 2, weiter umfassend ein pharmazeutisches Medium um die Polypeptidverbindung aufzunehmen, wobei das pharmazeutische Medium mindestens eines ist, ausgewählt aus der Gruppe, bestehend aus: einer wässrigen Lösung, Suspension, Dispersion, Salbe, Balsam, Gel, Creme, Lotion, Spray oder Paste.

4. Wundheilungszusammensetzung nach einem der Ansprüche 1 bis 3 zur Verwendung in der Heilung eine Hautwunde.

5. Wundheilungszusammensetzung zur Verwendung nach Anspruch 4, wobei die Polypeptidverbindung in einer Konzentration von 10 µg/ml bis 3 mg/ml in dem pharmazeutischen Medium formuliert ist.

6. Wundheilungszusammensetzung zur Verwendung nach Anspruch 4, wobei die Polypeptidverbindung in einer Konzentration von 30 µg/ml bis 500 µg/ml in dem pharmazeutischen Medium formuliert ist.

7. Wundheilungszusammensetzung zur Verwendung nach Anspruch 4, wobei die Zusammensetzung alle 6 bis 72 Stunden auf die Wunde aufgetragen wird.

8. Wundheilungszusammensetzung zur Verwendung nach Anspruch 4, wobei die Zusammensetzung alle 24 bis 48 Stunden auf die Wunde aufgetragen wird.

## Revendications

1. Composition de cicatrisation de plaie comprenant :
un composé polypeptidique comportant une chaîne polypeptidique hsp90α, dans laquelle la chaîne polypeptidique comprend la séquence d'acides aminés EEKEDKEEEKEKEEKESEDKPEIEDVGSDEEEEKKDGDKKKKKKIKEKYIDQEE (Identificateur de séquence No :1).

2. Composition de cicatrisation de plaie comprenant :
un composé polypeptidique comportant une chaîne polypeptidique hsp90α, dans laquelle la chaîne polypeptidique comprend la séquence d'acides aminés SDEEEEKKDGDKKKKKKIKEKYIDQEE (Identificateur de séquence No : 2).

3. Composition de cicatrisation de plaie selon la revendication 1 ou 2, comprenant, en outre, un milieu pharmaceutique afin de supporter le composé polypeptidique, dans laquelle le milieu pharmaceutique est au moins un élément sélectionné dans le groupe constitué par : une solution aqueuse, une suspension, une dispersion, un baume, un onguent, un gel, une crème, une lotion, un produit pulvérisé ou une pâte.

4. Composition de cicatrisation de plaie selon l'une quelconque des revendications 1 à 3 destinée à être utilisée pour la cicatrisation de plaie cutanées.

5. Composition de cicatrisation de plaie destinée à être utilisée selon la revendication 4, dans laquelle le composé polypeptidique est formulé avec une concentration de 10 µg/ml à 3 mg/ml dans ledit milieu pharmaceutique.

6. Composition de cicatrisation de plaie destinée à être utilisée selon la revendication 4, dans laquelle le composé polypeptidique est formulé avec une concentration de 30 µg/ml à 500 µg/ml dans ledit milieu pharmaceutique.

7. Composition de cicatrisation de plaie destinée à être utilisée selon la revendication 4, dans laquelle la composition est appliquée sur la plaie toutes les 6 à 72 heures.

8. Composition de cicatrisation de plaie destinée à être utilisée selon la revendication 4, dans laquelle la composition est appliquée sur la plaie toutes les 24 à 48 heures.
